# EUROPEAN PATENT SPECIFICATION

(11) **EP 1 364 631 B1**
(45) Date of publication and mention of the grant of the patent: **06.04.2005**
(21) Application number: 03011650.3
(22) Date of filing: 22.05.2003
(51) Int. Cl.: A61F 2/46

(54) **Test system for femoral prosthesis**
Testsystem für Femurprothese
Système d'essai pour prothèse fémorale

(30) Priority: 23.05.2002 FR 0206292
(43) Date of publication of application: 26.11.2003
(73) Proprietor: Zimmer GmbH, 8404 Winterthur (CH)
(72) Inventor: Le Beguec, Pierre, 35000 Rennes (FR)
(74) Representative: Manitz, Finsterwald & Partner GbR

(56) References cited:
- EP-A- 0 163 121
- EP-A- 0 931 524
- EP-A- 0 978 262
- WO-A-92/03993
- WO-A-96/00539
- US-A- 5 766 261

## Description

The invention relates to a test system for the selection of a prosthesis suitable for insertion as a so-called press fit effect femur prosthesis, for the cement-free implantation in a femur bone according to the preamble of claim 1.

In the field of hip joint prostheses two large groups of cement-free femur prostheses are known.

There are on the one hand so-called first implantation femur prostheses which are used on the patient for the first time and on the other hand so-called revision femur shafts which, as their name says, can replace an implant which has failed.

In both cases the principle aim is to ensure a complete primary stability of the femur shaft.

In order to achieve this aim, i.e. to produce a firm mechanical connection between the prosthesis and the surrounding bone, there are several methods of which two are frequently used and indeed the use of a locked shaft and the already mentioned press fit concept.

For the press fit concept to be effective, two important conditions must be satisfied simultaneously and indeed the production of a contact surface between the bone and the implant and then a problem-free positioning of the prosthesis in this contact region.

In order to attain this goal a shaft of conical shape has numerous advantages and indeed the production of a stabilizing horizontal force which ensures an improved distribution of the loads to the contact surface and indeed above all when it is of conical shape, which simultaneously facilitates the positioning and makes an adjustment possible.

Conical means not necessarily that the cross-section is a circle. It could also be ovoid or rectangular with generously rounded corners.

The positioning and the adjustment are however only possible with a conical shaft if one inserts a shaft during the implantation which has a conicity reserve available with reference to the conical seat in the bone, since if this is not the case a secondary sinkage of even a small amount could bring about a destabilization of the prosthesis.

WO 92/03993 A1 describes a modular trial hip replacement system having at least three trial stem portions of differing size and at least three trial body portions of different size.

The object of the invention is to select a shaft of the prosthesis a conicity reserve so that a secondary sinkage is avoided, having and to avoid an unnecessary distal end of the shaft since this might influence the surrounding bone and give the necessity to cut more bone away at a later subsequent implantation of a revision prosthesis.

The invention is based on the idea that one must use the distal part of the conical region of the prosthesis to obtain a conicity reserve, with it being taken into account that this conical region is of constant height, independently of the location of the conical seat, which is generated when using a rasp or reamer of adequate conicity. It is clear that with a tool of given conicity the seat achieved in the bone will depend with its depth on the morpho type of the bone.

In order to satisfy these two conditions a precise choice of the prosthesis to be inserted must therefore be effected.

This is naturally not a simple matter and indeed especially when replacing a loosened, so-called press fit prosthesis, with the choice of a prosthesis matched to the anatomical situation being an important working step, since the main aim is indeed to achieve a problem-free primary stability, with preference being given to the choice of a short shaft.

The principal aim of the invention mentioned above is achieved by a test system having the features of claim 1.

In order to make the system more sensitive third, fourth and further shafts can be foreseen, all shafts having a smaller step of maximum diameter D with reference to the precedent shaft and each shaft having the distal region of its cone congruent overlapped with the proximal region of the cone of the precedent shaft with the smaller maximum diameter D.

Preferred embodiments of the invention are set forth in the subordinate claims and in the following description.

The invention furthermore relates to the features which result from the following description and which are considered individually or in all their possible technical combinations.

This description, which does not count as a limiting example, and in which reference is made to the accompanying drawings, serves to illustrate how the invention can be carried out. In the drawing there are shown:
- Fig. 1: a schematic sectional view of a femur bone and of a first test prosthesis,
- Fig. 2: a schematic sectional view of the same femur bone and a second test prosthesis with a larger diameter which permits a conicity reserve, but with a length which is identical to the preceding test prosthesis but which proves to be too high having regard to the morpho type.
- Fig. 3: a schematic sectional view of a femur bone and a third prosthesis with the same diameter as the preceding prosthesis and with the same conicity reserve, but with a much shorter length than the preceding one in order to take account of the height of the morpho type.

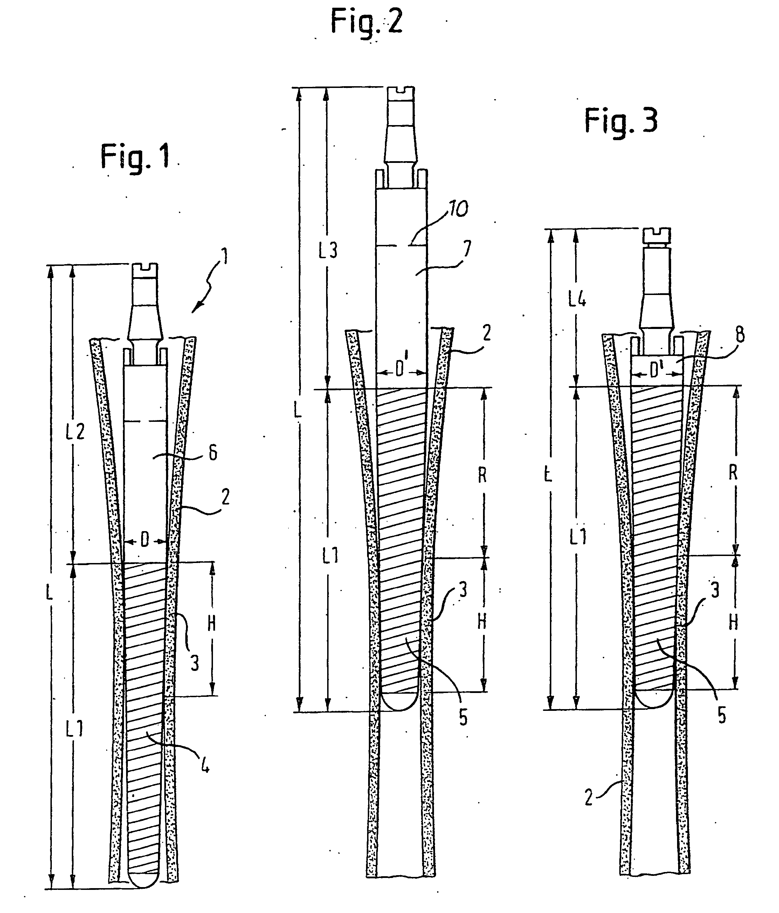

Before discussing the test prosthesis system 1 shown in the Figures, it should first be pointed out that the femur bone shown has previously been machined with a rasp or reamer to produce a conical seat in the bone. The precise vertical position of the conical recess machined within the bone relative to the resection surface at the top of the femur bone is not known. The resection surface is the surface shown at the top of the femur bone in Figs. 1 to 3. The surgeon using a rasp or reamer can, however, determine when the rasp or reamer has ceased to cut easily. This indicates that the soft material at the inner side of the femur bone has been removed and that the rasp or reamer has entered into contact with the hard material of the cortex.

It should also be pointed out that the rasp or reamer used to carry out this action has the same cone angle as each of the conical distal regions of the shafts of the test prosthesis which will now be explained in more detail.

Thus, one problem is that one does not know where the rasp or reamer has actually seated during the machining of the femur bone because the bone opens out in trumpet-like manner, even if the divergence of the femur bone towards the resection surface is relatively small. Accordingly, one also does not know where the actual prosthesis will sit which corresponds to the rasp or reamer which has been used.

Furthermore, the actual prosthesis should only project so far into the bone that a conical supporting surface in the bone is achieved which supports the prosthesis, with the prosthesis simultaneously also having a conical reserve length R which prevents secondary sinkage. That is to say, if the prosthesis tends to sink vertically downwardly in the bone, there is a conical region above the region initially engaging the bone which comes into a seating engagement with the conical recess in the bone and prevents further sinkage. If the prosthesis is penetrating the bone too much below the matching surface this might affect the bone which might be foreseen as support for subsequent later revision prosthesis.

The test prosthesis system 1 shown in the Figures is intended to permit a first choice of a cement-free femur prosthesis in accordance with the so-called press fit insertion method in a femur bone 2, in an intermediate region 3, with the press fit effect preferably being obtained at the level of the intermediate region 3, which corresponds to a vertical range H in the femur bone 2.

The prosthesis apparatus 1 illustrated in the Figures as an explanatory embodiment consists of a set of at least first and second shafts having first and second conical distal shaft portions 4, 5. The first distal shaft portion 4 has a first cone angle and a first maximum diameter D. The second conical distal shaft portion 5 has a second cone angle equal to the first cone angle and a second maximum diameter (shown with D in Fig. 2) which is greater than the first maximum diameter D (shown in Fig. 1). It is important to note that the first conical distal region 4 has a proximal region congruent to (i.e. overlapping in size and shape) with a distal region of said second conical distal region 5. The distal conical regions of the distal shaft portions 4, 5 each. preferably have the same length L1. In the example shown in Fig. 3 the conical distal portion of the shaft shown there is identical to the conical distal shaft portion 5 shown in Fig. 2, which is why it is also given the same reference numeral in Fig. 3. The conical distal regions are extended by cylindrical proximal parts 6, 7, 8. The diameters D, D' of the cylindrical proximal parts preferably correspond in each case to that of the associated conical distal parts. The respective lengths L2, L3, L4 of the cylindrical proximal parts 6, 7, 8 can however be different.

In the present case the prosthesis system consists of a set of two distal shaft portions 4, 5 of conical shape and with maximum diameters of 16 mm and 18 mm respectively. This maximum diameter is measured at the upper end of the conical region as seen in the drawing. The length L1 of the common conical region amounts to 110 mm in each case. The two distal shaft portions 4, 5 are complemented by proximal cylindrical parts 6, 7, 8 having lengths L2, L3, L4 of 90 mm, 90 mm and 30 mm respectively. Cylindrical part L2 has the same diameter as the maximum diameter of the distal shaft portion 4 and the cylindrical parts L3 and L4 have the same diameter as the maximum diameter of the distal shaft portion 5. In this way, three different test prostheses 1 are obtained as shown in Figs. 1, 2 and 3. The shaft of Fig. 1 has a length L of 200 mm and a maximum diameter D of 16 mm. The shaft of Fig. 2 has a length L of 200 mm and a maximum diameter D' of 18 mm and the shaft of Fig. 3 has a length L of 140 mm and a maximum diameter D of 18 mm. The first shaft in Fig. 1 with the proximal region of its cone 4 has an overlap with the distal region of the cone 5 of the second shaft in Figure 2 and Figure 3 for a length H, which corresponds to an estimated bone seat.

In accordance with one variant each of the shafts consists of two elements of which one is formed by the conical distal part and the other by the cylindrical proximal part, with connection means permitting their connection to one another so that modular combinations are possible.

In accordance with another feature of the invention a marking is provided between the conical part of the shaft and its cylindrical part in order to recite the upper range of the conicity reserve obtained with a specific shaft. As the average cone angle of the bone in the diaphysial region is rather small, such marking is helpful.

The method of using the above-described prosthesis system lies in determining, by sequential tests in the femur bone 2, the diameter D of the shaft 4, 5 which is to be used, on the one hand so that it makes contact in the press fit region 3 of the femur, preferably with its conical distal part, and, on the other hand, in selecting the length L2, L3, L4 of the cylindrical proximal part 6, 7, 8 for a diameter D of the selected shaft 4, 5 so that the height of the morpho type is observed, with the combination provided in this manner making it possible for the test prosthesis to have available a conicity reserve R relative to the femur bone 2 corresponding to that which the actual prosthesis corresponding to the test prosthesis has available when inserted, with the height of the morpho type being observed.

Thus, each test prosthesis consists of a proximal part having a cylindrical shape with a diameter D which merges into a conical distal part having a length L1, with the conical distal part having a maximum diameter D which is preferably equal to the proximal cylindrical part.

Each of the cylindrical proximal parts can carry a scale which enables the length of the cylindrical proximal part extending below the resection surface to be read off by comparison with the scale.

The minimum number of shafts which is required to put the invention into practice is two.

If, as the comparison of the Figures shows, one determines in a first test that the prosthesis which consists of the shaft 4 and the cylindrical part 6 having the maximum diameter of 16 mm is not suitable, because the shaft 4 does not contact the diaphysial region with its distal part but rather with its proximal part, so that no adequate conicity reserve is present, then this combination of distal shaft portion 4 and proximal cylindrical portion 6 has to be rejected. So a first test is made to measure the penetration depth of the first shaft with reference to the bone.

In a second test, as shown in Fig. 2, one can see, in contrast, that by using a distal shaft portion 5 with a maximum diameter of 18 mm the conical distal shaft portion 5 makes contact in the diaphysial region 3, as intended. By knowing the overlap region and the position of the first shaft with a reference mark 10 on the second shaft, it can be easily controlled with reference to the bone, whether the overlap region of the second shaft is at the same depth; and if it is so, the second shaft fits the bone with the distal part of its cone 5 having enough conicity reserve R to prevent secondary sinking. The proximal cylindrical part or portion 7 has the same length L3 of 90 mm as the previously used proximal cylindrical part or portion 6 and a maximum diameter of 18 mm.

Thus, in the second case, one can also see that the use of a larger diameter while retaining the same height L3 of 90 mm of the cylindrical part 7 leads to a larger height of the unit relative to the morpho type.

In Fig. 3 one now sees, in the third (optional) test, that the cylindrical part 7 with a length L3 of 90 mm, as above, can be substituted by a cylindrical part 8 with a length L4 of 30 mm, so that one obtains a test prosthesis of a total height of 140 mm which permits the morpho type to be observed.

Strictly speaking, the third shaft shown in Fig. 3 is not necessary. For example, if the proximal region of the shaft shown in Fig. 2 is provided with a scale, then the vertical position of the resection surface on the scale can be read off and the actual prosthesis can be selected based on this reading and on the conical size and shape of the conical distal part of the second shaft shown in Fig. 2. Instead of providing a scale it is also possible, for example, to measure the length of the cylindrical proximal part projecting above the resection surface. It is noted that the actual prosthesis has a distal conical region having the same cone angle and maximum diameter as the conical distal region of the appropriate test prosthesis, in the example given the conical distal region of the test shaft of Fig. 2, and has a length relative to the resection surface necessary to ensure equality of the length of the patient's limbs.

The above named dimensions serve, both with regard to the length and also with regard to the diameter, as an example and could also be selected differently.

In the same way one can also imagine a test apparatus which is offered in the form of a case containing a larger number of shafts in a specific range of lengths and diameters, i.e. a modular design.

After the suitable distal shaft portion has been selected a proximal element is fitted to it, which is likewise a test element, in order to form a complete test prosthesis which corresponds to the final prosthesis.

In order to understand the significance of the modularity which permits the provision of a conicity reserve one must, for example, know that a test prosthesis with a length of 200 mm and a diameter of 16 mm and a short prosthesis with a length of 140 mm, but with a diameter of 18 mm, have a common anchoring zone and this is located in a conical region of a short shaft in the distal position and in the conical region of a long shaft in the proximal position, which is why one aims at a short shaft.

This signifies that for a certain diameter of the femur bone one must consider replacing a long shaft with a shorter shaft but with a larger diameter.

This possibility has only advantages since, if one makes the selection of the short shaft under such circumstances, one provides the conicity reserve that is aimed at and simultaneously also implants a shorter shaft and indeed without supplementary drilling out.

The possibility of proceeding in this way is only possible if one has available a modulatable test prosthesis in accordance with the invention.

The question arises as to what is the smallest number of parts required to realise the test system in accordance with the invention. The answer is that one requires a minimum of two shafts with distal conical shaft portions 4 and 5 of the same length and the same angle but of different maximum diameters of, for example, 16 mm and 18 mm, creating an overlap, which corresponds to a wanted seat length H in the bone.

In this example, the distal shaft portion 4 with the smallest maximum diameter should be mated to or formed in one piece with a proximal shaft portion 6 of a relatively long length, e.g. 90 mm. The distal shaft portion 5 with the largest maximum diameter should be mated to or formed in one piece with a proximal cylindrical portion 8 also of a relatively long length, e.g. 90 mm.

In addition a third test element, which is not essential but convenient, can comprise a distal shaft portion 5 with the largest maximum diameter and can be mated to or formed in one piece with a proximal cylindrical portion 7 of a relatively short length, e.g. 30 mm.

If the distal shaft portions 4 or 5 are releasably connected to the proximal cylindrical portions 6, 7 and 8 then in a preferred design it is sufficient to provide just two distal shaft portions 4 and 5 and a total of just three proximal cylindrical portions; a long one 6 for the distal shaft portion 4 with the smallest maximum diameter and a long one 7 and a short one 8 for the distal shaft portion 5 with the largest maximum diameter. Naturally, the distal shaft portion 4 can be formed in one piece with the proximal cylindrical portion 6 and the distal shaft portion 5 can be formed as a module connectable to either the long cylindrical portion 7 or the short cylindrical portion 8.

The invention will now once again be described with reference to the concepts which are important in practice

Since at least two test prostheses, i.e. at least two test shafts, are provided having the same cone angle in the conical distal region, but with a different maximum diameter of the conical distal region, it is possible to determine precisely where the conical region which has been machined in the femur bone is situated with regard to its vertical position. In this connection it is important that the first conical distal region of the first shaft has a proximal region which is congruent to a distal region of the conical distal region of the second shaft. By congruent is meant that the proximal portion of the conical distal region of the first shaft overlaps in size and shape with a distal region of the conical distal portion of the second shaft. By trying these two test shafts in turn it is possible to determine whether the conical supporting surface is located in the overlapping region and, if so, how large the reserve is for the test shaft with the larger diameter.

Thus, in the example of Fig. 1, using the test shaft with the smaller maximum diameter of the conical distal region, it can be seen that the test shaft sinks well down into the bone. If this test shaft is then replaced with the test shaft of Fig. 2 it can be seen that the test shaft now seats much higher up in the femur bone. In each case the seat occurs in the conical region of the bone prepared by the rasp or reamer. Since the first and second shafts have congruent overlapping portions, a comparison of the penetration depth of the congruent overlapping portions enables the precise vertical position of the conical region of engagement of the bone with the test prosthesis to be determined. The comparison is very simple if the second shaft has a mark, which shows the upper level of the first shaft, when the congruent overlapping of the conical regions occurs. This also makes it possible for the surgeon to determine precisely which length the actual prosthesis should have in order, on the one hand, to seat reliably in the press-fit region determined by the conically machined recess in the bone and still have a conical reserve, i.e. a conical region above the region of engagement with the conical recess in the bone, so that, if a sinkage occurs, there is still engagement within the bone over the full length of the conical distal region of the actual prosthesis.

It is also possible to provide further shafts having respective conical distal regions with the same cone angle but with a larger (or smaller) maximum diameter. In each case the conical distal region of each further shaft should have a distal conical region congruent to a proximal conical region of the next preceding shaft. If a third test shaft is provided having a conical distal region with a maximum diameter of 20 mm, so that its distal region engages into the proximal reserve region of the test prosthesis with 18 mm of diameter, then, in the example of Fig. 2, this even larger test prosthesis would hardly wedge into the femur bone, but could instead still be easily pivoted slightly within the femur bone because it seats not over a considerable conical length, but rather only at its bottom end. The surgeon would thus know that the test prosthesis, i.e. the second shaft with D = 18 mm, would be best suited when its length in the cylindrical part is matched appropriately to the resection surface.

In this manner it is possible to determine the position of the conical seat of the bone, to select a corresponding test prosthesis or test shaft, or to assemble a test prosthesis or test shaft if a modular construction is present (separate cylindrical proximal parts and conical distal parts which can be put together) and to check the seat, including the conical reserve, in order to later select an actual prosthesis which has the appropriate conical reserve and the appropriate overall length.

## Claims

1. A test system (1) for the selection of a prosthesis for insertion as a so-called press-fit-effect femur prosthesis, for cement-free implantation into a femur bone (2) in a region (3) in which the press fit effect is preferably obtained, this region (3) corresponding to a diaphysial region, the vertical position of which differs depending on the morpho type, the test system consisting of a set of at least first and second shafts, the first shaft having a first conical, distal region (4), said first conical distal region (4) having a first cone angle and a first maximum diameter (D), as well as a proximal region (6) having a first vertical height (L2) and the second shaft having a second conical distal region (5), said second conical distal region (5) having a second cone angle equal to said first cone angle and a second maximum diameter (D') greater than said first maximum diameter (D), as well as a proximal region (7) having a second vertical height (L3), said first conical distal region (4) having a proximale region of a certain length (H) congruent with a distal region of said second conical distal region (5),
**characterized in that**
the second shaft has a mark (10), which shows the upper level of the first shaft, when congruent overlapping of the congruent conical regions of said length (H) occurs.

2. A test system in accordance with claim 1, wherein said conical distal region (5) of the second shaft is of at least substantially the same lengt (L1) as said conical distal region (4) of said first shaft.

3. A test system in accordance with claim 1 or claim 2, wherein said first shaft has an overall length (L) and said second shaft has an overall length (L) at least substantially equal to said overall length (L) of said first shaft.

4. A test system in accordance with any one of the preceding claims, wherein said proximal region of said first shaft has a first length and said proximal region of said second shaft has a second length equal to said first length.

5. A test system in accordance with any one of the preceding claims and including at least one further shaft having a cone angle equal to said first cone angle and a maximum diameter greater than a maximum . diameter of the next preceding shaft, said conical distal region of said further shaft having a distal region congruent to a proximal region of the next preceding shaft.

6. A test system in accordance with claim 5, said at least one further shaft having an overall length equal to an overall length of a next preceding shaft.

7. A test system in accordance with claim 5 having an overall length shorter than an overall length of said next preceding shaft.

8. A test system in accordance with any one of the preceding claims, the proximal regions (6, 7) of each said shaft having a length scale.

9. A test system in accordance with any one of the preceding claims, wherein the conical distal region (4, 5) of each said shaft is integral with the respective proximal region (6, 7) the same shaft.

10. A test system in accordance with any one of the preceding claims 1 to 8, wherein at least one of said shafts comprises a conical distal region. and a proximal region separable therefrom.

11. A test system in accordance with any one of the preceding claims1 to 8, wherein said set comprises a plurality of proximal shaft modules interchangeably connected with a plurality of conical distal modules.

12. A test system in accordance with any one of the preceding claims, **characterized in that** it consists of a set of shafts having distal conical shaft portions (4, 5) with maximum diameters of 16 mm and 18 mm and a length (L1) of the common conical region of 110 mm and of proximal cylindrical portions (6, 7, 8) with diameters of D = 16 mm and D' = 18 mm respectively and lengths (L2, L3, L4) of 90 mm, 90 mm and 30 mm in order to obtain a first test prosthesis (1) with a length (L) of 200 mm and a diameter (D) of 16 mm, a second test prosthesis with a length (L) of 200 mm and a diameter (D') of 18 mm and a third test prosthesis with a length (L) of 140 mm and a diameter (D') of 18 mm.

13. A test system in accordance with one of the claims 1 to 8 or 10 to 12, **characterized in that** each of the shafts consists of two elements, of which one is formed by a conical distal part (4, 5) and of which the other is formed by a cylindrical proximal part (6, 7, 8) with connection means permitting them to be connected together by allowing modular combinations.

14. A test system in accordance with any one of the preceding claims, **characterized in that** a marking is provided on each said shaft to recite the upper range of the conicity obtained with that shaft.

## Patentansprüche

1. Testsystem (1) zur Auswahl einer Prothese zum Einsetzen als eine so genannte Pressfiteffekt-Femurprothese zur zementfreien Implantation in einen Femurknochen (2) in einem Bereich (3), in dem der Pressfiteffekt bevorzugt erhalten wird, wobei dieser Bereich (3) einem Diaphysebereich entspricht, dessen vertikale Position sich abhängig von dem Morphotyp unterscheidet, wobei das Testsystem einen Satz aus zumindest einem ersten und zweiten Schaft umfasst, wobei der erste Schaft einen ersten konischen distalen Bereich (4), wobei der erste konische distale Bereich (4) einen ersten Kegelwinkel und einen ersten maximalen Durchmesser (D) besitzt, wie auch einen proximalen Bereich (6) mit einer ersten vertikalen Höhe (L2) aufweist, und der zweite Schaft einen zweiten konischen distalen Bereich (5), wobei der zweite konische distale Bereich (5) einen zweiten Kegelwinkel, der gleich dem ersten Kegelwinkel ist, und einen zweiten maximalen Durchmesser (D') aufweist, der größer als der erste maximale Durchmesser (D) ist, wie auch einen proximalen Bereich (7) mit einer zweiten vertikalen Höhe (L3) aufweist, wobei der erste konische distale Bereich (4) einen proximalen Bereich mit einer bestimmten Länge (H) besitzt, der mit einem distalen Bereich des zweiten konischen distalen Bereichs (5) übereinstimmt, **dadurch gekennzeichnet, dass** der zweite Schaft eine Markierung (10) besitzt, die das obere Niveau des ersten Schaftes zeigt, wenn die übereinstimmende Überlappung der übereinstimmenden konischen Bereiche der Länge (H) erfolgt.

2. Testsystem nach Anspruch 1, wobei der konische distale Bereich (5) des zweiten Schaftes zumindest im Wesentlichen dieselbe Länge (L1) wie der konische distale Bereich (4) des ersten Schaftes umfasst.

3. Testsystem nach einem der Ansprüche 1 oder 2, wobei der erste Schaft eine Gesamtlänge (L) besitzt und der zweite Schaft eine Gesamtlänge (L) besitzt, die zumindest im Wesentlichen gleich der Gesamtlänge (L) des ersten Schaftes ist.

4. Testsystem nach einem der vorhergehenden Ansprüche, wobei der proximale Bereich des ersten Schaftes eine erste Länge besitzt und der proximale Bereich des zweiten Schafts eine zweite Länge besitzt, die gleich der ersten Länge ist.

5. Testsystem nach einem der vorhergehenden Ansprüche, mit zumindest einem weiteren Schaft, der einen Kegelwinkel aufweist, der gleich dem ersten Kegelwinkel ist, und der einen maximalen Durchmesser aufweist, der größer als ein maximaler Durchmesser des nächsten vorangehenden Schaftes ist, wobei der konische distale Bereich des weiteren Schaftes einen distalen Bereich aufweist, der mit einem proximalen Bereich des nächsten vorausgehenden Schaftes übereinstimmt.

6. Testsystem nach Anspruch 5, wobei der zumindest eine weitere Schaft eine Gesamtlänge aufweist, die gleich einer Gesamtlänge eines nächsten vorangehenden Schaftes ist.

7. Testsystem nach Anspruch 5, wobei eine Gesamtlänge kürzer als eine Gesamtlänge des nächsten vorangehenden Schaftes ist.

8. Testsystem nach einem der vorhergehenden Ansprüche, wobei die proximalen Bereiche (6, 7) von jedem Schaft eine Längenskalierung besitzen.

9. Testsystem nach einem der vorhergehenden Ansprüche, wobei der konische distale Bereich (4, 5) von jedem Schaft einteilig mit dem jeweiligen proximalen Bereich (6, 7) an demselben Schaft ausgebildet ist.

10. Testsystem nach einem der Ansprüche 1 bis 8, wobei zumindest einer der Schäfte einen konischen distalen Bereich und einen davon trennbaren proximalen Bereich umfasst.

11. Testsystem nach einem der Ansprüche 1 bis 8, wobei der Satz eine Vielzahl proximaler Schaftmodule umfasst, die auswechselbar mit einer Vielzahl konischer distaler Module verbunden sind.

12. Testsystem nach einem der vorhergehenden Ansprüche, **dadurch gekennzeichnet, dass** es aus einem Satz von Schäften besteht, die distale konische Schaftabschnitte (4, 5) mit maximalen Durchmessern von 16 mm und 18 mm und einer Länge (L1) des gemeinsamen konischen Bereiches von 110 mm besitzen, und proximale zylindrische Abschnitte (6, 7, 8) mit Durchmessern von D = 16 mm bzw. D' = 18 mm und Längen (L2, L3, L4) von 90 mm, 90 mm und 30 mm umfassen, um eine erste Testprothese (1) mit einer Länge (L) von 200 mm und einem Durchmesser (D) von 16 mm zu erhalten, eine zweite Testprothese mit einer Länge (L) von 200 mm und einem Durchmesser (D') von 18 mm zu erhalten und eine dritte Testprothese mit einer Länge (L) von 140 mm und einem Durchmesser (D') von 18 mm zu erhalten.

13. Testsystem nach einem der Ansprüche 1 bis 8 oder 10 bis 12, **dadurch gekennzeichnet, dass** jeder der Schäfte aus zwei Elementen besteht, von denen eines durch ein konisches distales Teil (4, 5) und das andere durch ein zylindrisches proximales Teil (6, 7, 8) gebildet wird, wobei ein Verbindungsmittel zulässt, dass diese miteinander verbunden werden können, indem modulare Kombinationen möglich sind.

14. Testsystem nach einem der vorhergehenden Ansprüche, **dadurch gekennzeichnet, dass** eine Markierung an jedem Schaft vorgesehen ist, um den oberen Bereich der mit diesem Schaft erhaltenen Konizität darzustellen.

## Revendications

1. Système de test (1) pour la sélection d'une prothèse pour insertion telle qu'une prothèse de fémur dite à effet d'ajustement par serrage, pour une implantation sans ciment dans un fémur (2) dans une région (3) dans laquelle l'effet d'ajustement par serrage est de préférence obtenu, cette région (3) correspondant à une région diaphysaire, dont la position verticale diffère en fonction du type morphologique, le système de test étant constitué d'un ensemble d'au moins une première et une seconde tige, la première tige ayant une première région distale conique (4), ladite première région conique distale (4) ayant un premier angle conique et un premier diamètre maximum (D), ainsi qu'une région proximale (6) ayant une première hauteur verticale (L2) et la seconde tige ayant une seconde région distale conique (5), ladite seconde région distale conique (5) ayant un second angle conique égal audit premier angle conique et un second diamètre maximum (D') supérieur audit premier diamètre maximum (D), ainsi qu'une région proximale (7) ayant une seconde hauteur verticale (L3), ladite première région distale conique (4) ayant une région proximale d'une certaine longueur (H) en harmonie avec une région distale de ladite seconde région distale conique (5),
**caractérisé en ce que**
la seconde tige comporte une marque (10), qui montre le niveau supérieur de la première tige, lorsqu'un chevauchement adéquat des régions coniques adéquates de ladite longueur (H) se produit.

2. Système de test selon la revendication 1, dans lequel ladite région distale conique (5) de la seconde tige présente, au moins sensiblement, la même longueur (L1) que ladite région distale conique (4) de ladite première tige.

3. Système de test selon la revendication 1 ou la revendication 2, dans lequel ladite première tige a une longueur globale (L) et ladite seconde tige a une longueur globale (L) au moins sensiblement égale à ladite longueur globale (L) de ladite première tige.

4. Système de test selon l'une quelconque des revendications précédentes, dans lequel ladite région proximale de ladite première tige a une première longueur et ladite région proximale de ladite seconde tige a une seconde longueur égale à ladite première longueur.

5. Système de test selon l'une quelconque des revendications précédentes et comprenant au moins une autre tige ayant un angle conique égal audit premier angle conique et un diamètre maximum supérieur à un diamètre maximum de la tige immédiatement précédente, ladite région distale conique de ladite autre tige ayant une région distale en harmonie avec une région proximale de la tige précédente.

6. Système de test selon la revendication 5, ladite autre tige au moins ayant une longueur globale égale à une longueur globale d'une tige précédente.

7. Système de test selon la revendication 5 ayant une longueur globale plus courte qu'une longueur globale de ladite tige précédente.

8. Système de test selon l'une quelconque des revendications précédentes, les régions proximales (6, 7) de chaque dite tige ayant une échelle de longueur.

9. Système de test selon l'une quelconque des revendications précédentes, dans lequel la région distale conique (4, 5) de chaque dite tige est intégrée à la région proximale respective (6, 7) de la même tige.

10. Système de test selon l'une quelconque des revendications précédentes 1 à 8, dans lequel au moins l'une desdites tiges comprend une région distale conique et une région proximale pouvant être séparée de cette dernière.

11. Système de test selon l'une quelconque des revendications précédentes 1 à 8, dans lequel ledit ensemble comprend une pluralité de modules de tige proximaux reliés de manière interchangeable à une pluralité de modules distaux coniques.

12. Système de test selon l'une quelconque des revendications précédentes, **caractérisé en ce qu'**il est constitué d'un ensemble de tiges ayant des parties de tige coniques distales (4, 5) de diamètres maximaux de 16 mm et 18 mm et une longueur (L1) de la région conique commune de 110 mm et des parties cylindriques proximales (6, 7, 8) de diamètres respectifs de D = 16 mm et D' = 18 mm et des longueurs (L2, L3, L4) de 90 mm, 90 mm et 30 mm afin d'obtenir une première prothèse de test (1) d'une longueur (L) de 200 mm et d'un diamètre (D) de 16 mm, une deuxième prothèse de test d'une longueur (L) de 200 mm et d'un diamètre (D') de 18 mm et une troisième prothèse de test d'une longueur (L) de 140 mm et d'un diamètre (D') de 18 mm.

13. Système de test selon l'une des revendications précédentes 1 à 8 ou 10 à 12, **caractérisé en ce que** chacune des tiges est constituée de deux éléments, dont l'un est formé par une partie distale conique (4, 5) et dont l'autre est formé par une partie proximale cylindrique (6, 7, 8) avec des moyens de raccordement leur permettant d'être reliés en autorisant des combinaisons modulaires.

14. Système de test selon l'une quelconque des revendications précédentes, **caractérisé en ce qu'**un marquage est disposé sur chaque dite tige pour représenter la plage supérieure de la conicité obtenue avec cette tige.
